# EUROPÄISCHE PATENTSCHRIFT

(11) **EP 0 731 094 B1**
(45) Veröffentlichungstag und Bekanntmachung des Hinweises auf die Patenterteilung: **20.09.2000**
(21) Anmeldenummer: 96102714.1
(22) Anmeldetag: 23.02.1996
(51) Int. Cl.: C07D 231/12

(54) **Verfahren zur Herstellung von Pyrazolen**
Process for the preparation of pyrazoles
Procédé pour la préparation de pyrazoles

(30) Priorität: 07.03.1995 DE 19507915
(43) Veröffentlichungstag der Anmeldung: 11.09.1996
(73) Patentinhaber: BAYER AG, 51368 Leverkusen (DE)
(72) Erfinder: Wroblowsky, Heinz-Jürgen, Dr., 40764 Langenfeld (DE); Lantzsch, Reinhard, Dr., 42115 Wuppertal (DE)

(56) Entgegenhaltungen:
- EP-A- 0 526 281
- DE-A- 1 670 692
- DE-A- 2 310 185
- DE-B- 1 234 223
- US-A- 5 118 853
- CHEMISCHE BERICHTE, Bd. 93, Nr. 5, 1960, Seiten 1208-1211, XP002007265 H. BREDERECK ET AL:

## Beschreibung

Die Erfindung betrifft ein neues Verfahren zur Herstellung von Pyrazolen.

Es ist bekannt, daß man Pyrazole erhält, wenn man Hydrazine der Formel (A) mit 3-Dimethylaminoacrolein der Formel (B) gemäß dem folgenden Reaktionsschema umsetzt: 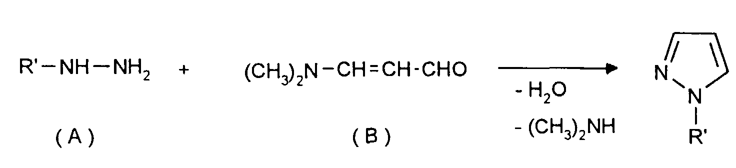 (vgl. hierzu DE-OS 1 670 692 oder Coll. czechoslov. chem. Commun. 23, 452 (1958)).

Dieses Verfahren hat jedoch den Nachteil, daß die Ausgangsstoffe der Formel (B) insbesondere im Hinblick auf ein technisches Verfahren nur in unbefriedigenden Ausbeuten erhältlich sind (vgl. z.B. o.g. Literaturstelle sowie Zh. Org. Khim 8, 1394 (1972) und US 5 118 853) und ihre notwendige Isolierung durch Destillation einen erheblichen zusätzlichen Aufwand bedeutet.

Ferner ist bekannt, daß Pyrazole erhalten werden können, wenn man z.B. Dimethylformamid mit Phosgen versetzt, das entstehende Formylierungsmittel der Formel (C) mit einem Enamin der Formel (D) umsetzt und schließlich das so erhaltene salzartige Zwischenprodukt der Formel (E) mit Hydrazinen der Formel (A) gemäß dem folgenden Reaktionsschema umsetzt: 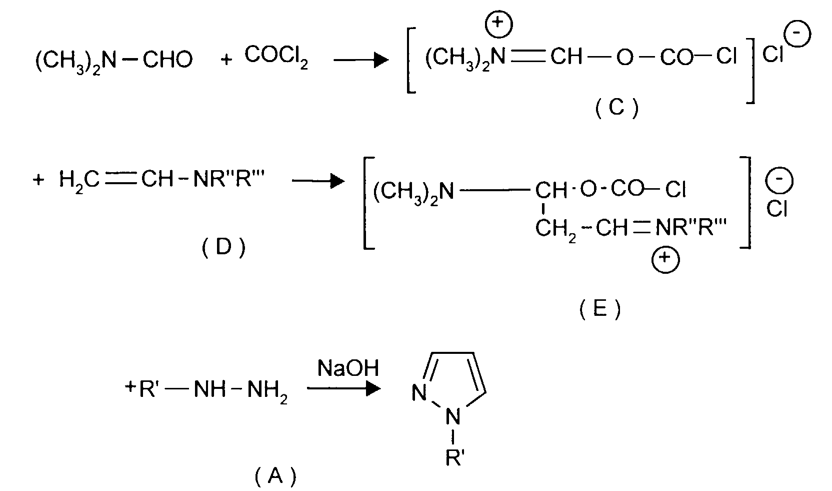 (vgl. hierzu DE-OS 1 234 223).

Dieses Verfahren hat jedoch den Nachteil, daß die Enamine der Formel (D) zum Teil schlecht zugänglich sind bzw. ein toxisches Potential besitzen. Außerdem bedeutet die Rückführung bzw. Entsorgung des beim Ringschluß frei werdenden Amins einen verhältnismäßig hohen Aufwand.

Es wurde nun gefunden, daß man Pyrazole der Formel (I) 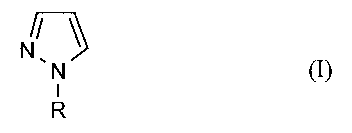 in welcher
- R: für Wasserstoff, geradkettiges oder verzweigtes C₁-C₄-Alkyl, gegebenenfalls einfach bis dreifach, gleich oder verschieden substituiertes C₃-C₆-Cycloalkyl, wobei als Substituenten Halogen, jeweils geradkettiges oder verzweigtes C₁-C₄-Alkyl, C₁-C₄-Alkoxy C₁-C₄-Halogenalkyl und C₁-C₄-Halogenalkyloxy in Frage kommen, oder gegebenenfalls einfach bis dreifach, gleich oder verschieden substituiertes Phenyl oder Benzyl, wobei jeweils als Substituenten Halogen, Cyano, Nitro, jeweils geradkettiges oder verzweigtes C₁-C₄-Alkyl, C₁-C₄-Alkoxy, C₁-C₄-Alkylthio, C₁-C₄-Halogenalkyl, C₁-C₄-Halogenalkoxy und C₁-C₄-Halogenalkylthio (mit jeweils 1 bis 9 gleichen oder verschiedenen Halogenatomen, insbesondere Fluor- und Chloratomen)
steht, erhält,
wenn man Propenylidenammoniumchlorid-Derivate der Formel (II) 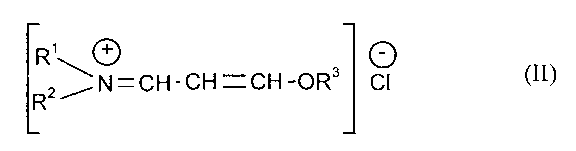 in welcher
- R¹ und R²: gleich oder verschieden sind und für geradkettiges oder verzweigtes C₁-C₄-Alkyl stehen und
- R³: für geradkettiges oder verzweigtes C₁-C₄-Alkyl steht,
mit Hydrazinen der Formel (III)

H₂N-NH-R (III)

in welcher
- R: die oben angegebene Bedeutung hat,
in Gegenwart von Alkalimetall- oder Erdalkalimetall-hydroxiden, -amiden, -alkoholaten, -carbonaten, -hydrogencarbonaten, Trialkylaminen oder Dialkylaminen und in Gegenwart von Alkoholen, Ethern, gegebenenfalls halogenierten Kohlenwasserstoffen oder Wasser als Verdünnungsmittel umsetzt.

Überraschenderweise können nach dem erfindungsgemäßen Verfahren Pyrazole der Formel (I) in sehr guten Ausbeuten und in hoher Reinheit erhalten werden, obwohl nach dem Stand der Technik erwartet werden mußte, daß diese Reaktion nur dann erfolgreich verläuft, wenn vor der Umsetzung die Propenylidenammoniumchloride der Formel (II) zu den entsprechenden freien Basen der Formel (IIa) 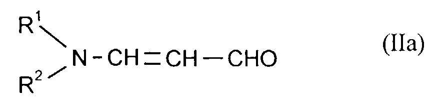 in welcher
- R¹ und R²: die oben angegebene Bedeutung haben,
hydrolisiert werden.

Die erfindungsgemäße Umsetzung hat somit den Vorteil einer günstigeren Verfahrensdurchführung bei gleichzeitiger Ausbeuteverbesserung, insbesondere im Hinblick auf das Gesamtverfahren, d.h. unter Einbeziehung der Vorproduktherstellung.

Nach dem erfindungsgemäßen Verfahren werden bevorzugt Pyrazole der Formel (I) hergestellt, in welcher
- R: für Wasserstoff, Methyl, Ethyl, n- oder i-Propyl, n-, i-, s- oder t-Butyl oder für jeweils gegebenenfalls einfach oder zweifach, gleich oder verschieden substituiertes Cyclopentyl oder Cyclohexyl steht, wobei als Substituenten Fluor, Chlor, Brom, Methyl, Ethyl, Trifluormethyl und Trifluormethoxy in Frage kommmen, oder für jeweils gegebenenfalls einfach bis dreifach, gleich oder verschieden substituiertes Phenyl oder Benzyl steht, wobei jeweils als Substituenten Fluor, Chlor, Cyano, Nitro, Methyl, Ethyl, n- oder i-Propyl, n-, i-, s- oder t-Butyl, Trifluormethyl, Trifluormethoxy, Trifluormethylthio und gegebenenfalls einfach oder zweifach durch Fluor, Chlor und/oder Methyl substituiertes Phenyl in Frage kommen.
Verwendet man beispielsweise 3-Ethoxypropenylidendimethylammonium-chlorid und Hydrazinhydrat als Ausgangsstoffe, so kann der Reaktionsablauf des erfindungsgemäßen Verfahrens durch das folgende Formelschema skizziert werden:

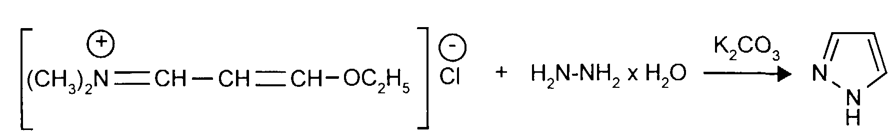

Die beim erfindgungsgemäßen Verfahren als Ausgangsstoffe zu verwendenden Propenylidenammoniumchlorid-Derivate sind durch die Formel (II) allgemein definiert. In der Formel (II) stehen R¹ und R² vorzugsweise für Methyl, Ethyl, n- oder i-Propyl, n- oder i-Butyl. R³ steht vorzugsweise für Methyl, Ethyl, n- oder i-Propyl, n- oder i-Butyl.

Die Propenylidenammoniumchlorid-Derivate der Formel (II) sind prinzipiell bekannt (vgl. z.B. US-5 118 953).

Sie werden erhalten, indem man Dialkylformamide der Formel (IV) 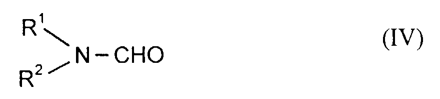 in welcher
- R¹ und R²: die oben angegebene Bedeutung haben,
mit Säurechloriden, wie beispielsweise Oxalylchlorid, Phosgen, Phosphoroxychlorid oder Thionylchlorid in Gegenwart eines Verdünnungsmittels wie beispielsweise Methylenchlorid bei Temperaturen zwischen 0°C und 50°C umsetzt und die entstehenden Zwischenprodukte der Formel (V) 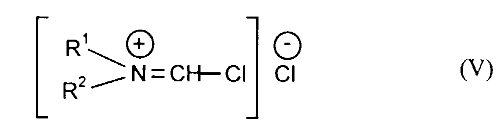 in welcher
- R¹ und R²: die oben angegebene Bedeutung haben,
ohne Isolierung direkt mit Vinylethern der allgemeinen Formel (VI)

CH₂=CH-OR³ (VI)

in welcher
- R³: die oben angegebene Bedeutung hat,
umsetzt.

Die Propenylidenammoniumchlorid-Derivate der Formel (II) fallen dabei vorzugsweise mit 0,5 bis 1,0 Äquivalenten zusätzlich gebundenen Chlorwasserstoff an (vgl. auch die Herstellungsbeispiele).

Die Dimethylformamide der Formel (IV) und die Vinylether der Formel (VI) sind allgemein bekannte Verbindungen der Organischen Chemie.

Die außerdem beim erfindungsgemäßen Verfahren als Ausgangsstoffe zu verwendenden Hydrazine sind durch die Formel (III) allgemein definiert. In der Formel (III) hat R bevorzugt bzw. besonders bevorzugt diejenige Bedeutung, die bereits oben im Zusammenhang mit der Beschreibung der erfindungsgemäß herzustellenden Pyrazole der Formel (I) als bevorzugt bzw. besonders bevorzugt für R angegeben wurde.

Die Hydrazine der Formel (III) sind allgemein bekannte Verbindungen der Organischen Chemie. Das unsubstituierte Hydrazin (R = H) wird vorzugsweise in Hydratform eingesetzt.

Das erfindungsgemäße Verfahren wird in Gegenwart von Alkalimetall- und Erdalkalimetall-hydroxiden, -amiden, -alkoholaten, -carbonaten oder -hydrogencarbonaten, Trialkylaminen oder Dialkylaminen durchgeführt. Bevorzugt sind Natriumhydroxid, Kaliumhydroxid, Natriumamid, Natriummethylat, Natriumethylat, Kalium-tert.-butylat, Natriumcarbonat, Kaliumcarbonat, Calciumcarbonat, Kaliumhydrogencarbonat, Natriumhydrogencarbonat, Trimethylamin, Triethylamin, Tributylamin, Dibutylamin und das dem eingesetzten Dialkylformamid der Formel (IV) entsprechende Dialkylamin.

Das erfindungsgemäße Verfahren wird in Gegenwart von Alkoholen, Ethern, gegebenenfalls halogenierten Kohlenwasserstoffen oder Wasser als Verdünnungsmittel durchgeführt.

Als Verdünnungsmittel kommen vorzugsweise Ethanol, Diethylether, Diisopropylether, Methyl-tert.-butyl-ether, Tetrahydrofuran, Dichlormethan, Chloroform und Tetrachlormethan in Betracht.

Die Reaktionstemperaturen können bei der Durchführung des erfindungsgemäßen Verfahrens in einem größeren Bereich variiert werden. Im allgemeinen arbeitet man bei Temperaturen zwischen 0°C und 150°C, vorzugsweise bei Temperaturen zwischen 20°C und 80°C.

Zur Durchführung des erfindungsgemäßen Verfahrens setzt man pro Mol Propenylidenammoniumchlorid-Derivat der Formel (II) im allgemeinen 1 bis 3 Mol, vorzugsweise 1 bis 1,5 Mol Hydrazin der Formel (III) und 1 bis 3 Mol, vorzugsweise 1 bis 1,5 Mol Base ein.

Die Reaktionskomponenten können bei der Durchführung des erfindungsgemäßen Verfahrens in beliebiger Reihenfolge eingesetzt werden.

In einer besonderen Ausführungsform des erfindungsgemäßen Verfahrens ist es auch möglich, die Ausgangsprodukte der Formel (II) ohne Isolierung nach ihrer Herstellung direkt weiter unzusetzen (vgl. auch die Herstellungsbeispiele).

Die Aufarbeitung kann auf übliche Art und Weise durchgeführt werden (vgl. auch die Herstellungsbeispiele).

Die nach dem erfindungsgemäßen Verfahren herzustellenden Pyrazole der Formel (I) können als Zwischenprodukte zur Herstellung von biologisch aktiven Verbindungen, beispielsweise von Insektiziden verwendet werden (vgl. z.B. EP-A-438 690).

### Herstellungsbeispiele:

### Beispiel 1

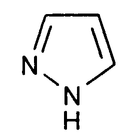

27,2 g (0,54 Mol) Hydrazinhydrat werden in 300 ml Ethanol gelöst und nach Zugabe von 75,2 g (0,54 Mol) Kaliumcarbonat die Lösung von 104,5 g (0,54 Mol) 3-Ethoxypropenylidendimethylammoniumchlorid-hydrochlorid (Bsp.-II-1) in 50 ml Ethanol bei 25 bis 35°C zugetropft. Nach vollendeter Zugabe wird über Nacht bei Raumtemperatur nachgerührt. Anschließend wird filtriert, mit Methyl-tert.-butyl-ether nachgewaschen und das Filtrat eingeengt. Zur Entfernung der Salze wird der Rückstand mit Methyl-tert.-butyl-ether ausgerührt, die unlöslichen Salze abfiltriert und das Filtrat eingedampft.
Das so erhaltene Rohprodukt wird im Vakuum bei 0,5 mbar und einer Badtemperatur von 100 bis 120°C destilliert.
Man erhält 31,0 g (84,4% der Theorie) Pyrazol mit einer Reinheit von 98,5%.
¹H-NMR(ppm in d⁶-DMSO): 6.261 (tr, 1H); 7.610 (s, 2H); 12.843 (broad s, 1H).

### (Ohne Isolierung des Ausgangsproduktes der Formel II)

36,5 g (0,5 Mol) Dimethylformamid werden in 250 ml Dichlorethan vorgelegt und bei 0°C 22 g (0,22 Mol) Phosgen eingeleitet. Anschließend tropft man bei 0°C 20 g (0,2 Mol) Butylvinylether zu, läßt auf Raumtemperatur kommen und erwärmt dann 15 Minuten auf 70°C. Anschließend läßt man abkühlen, gibt 80 g Eis hinzu und tropft 70 ml gesättigte Kaliumcarbonat-Lösung hinzu. Bei einer Badtemperatur von 100°C destilliert man das Dichlorethan ab und tropft dann bei 70°C 10 g (0,2 Mol) Hydrazinhydrat zu. Nach vollendeter Zugabe erwärmt man 10 Minuten auf 95°C und läßt dann abkühlen.
Nach Aufarbeitung der Reaktionslösung erhält man 10,9 g (80,3% der Theorie) an Pyrazol.

Gemäß Beispiel 1 bzw. entsprechend den allgemeinen Verfahrensangaben werden die folgenden Pyrazole der Formel (I) 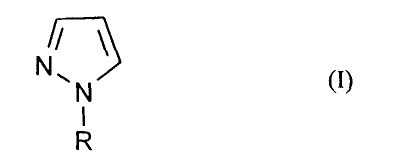 erhalten:

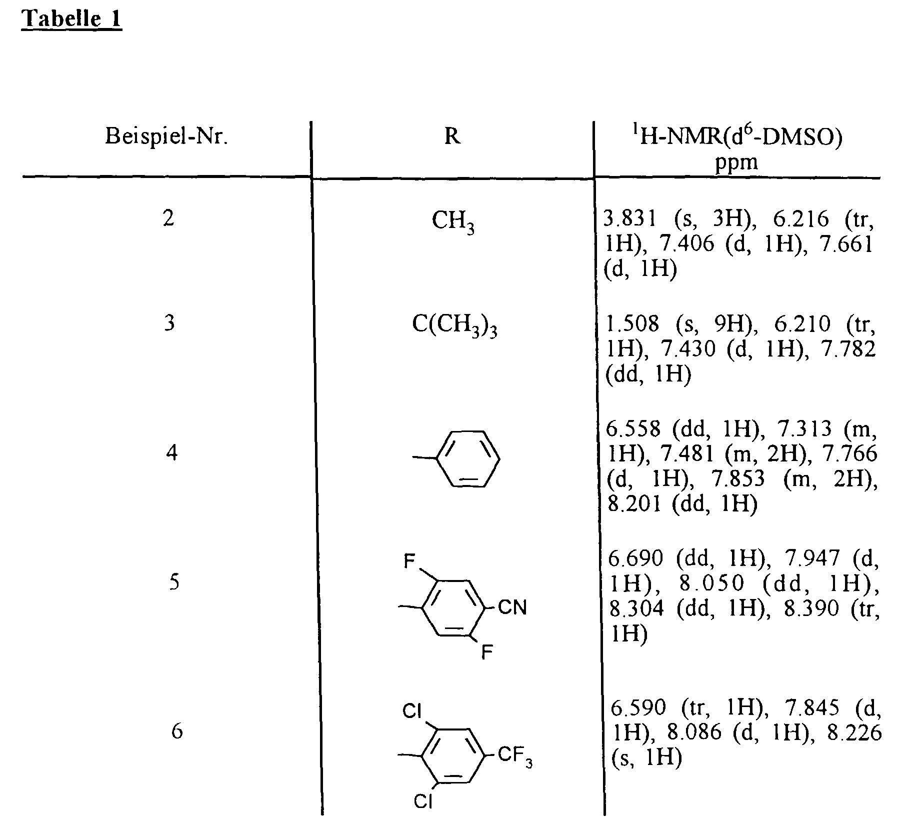

### Herstellung der Ausgangsprodukte der Formel (II)

### Beispiel (II-1)

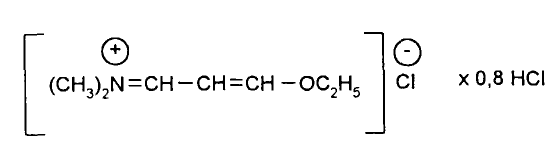

Unter Schutzgas werden 109,5 g (1,5 Mol) Dimethylformamid mit 800 ml Methylenchlorid versetzt und innerhalb von 3 Stunden bei 5 bis 10°C 215 g (1,7 Mol) Oxalylchlorid zugetropft. Man läßt auf Raumtemperatur kommen und dosiert innerhalb von 45 Minuten bei 25 bis 28°C 121 g (1,68 Mol) Ethylvinylether zu der weißen Suspension (stark exotherm). Die rotbraune Reaktionsmischung wird anschließend 30 Minuten bei 37 bis 40°C gerührt und nachfolgend das Methylenchlorid bei 30 mm Hg und 45°C Badtemperatur abdestilliert.
Man erhält 285,6 g (95,2% der Theorie) rohes 3-Ethoxypropenylidendimethylammoniumchlorid-hydrochlorid, das direkt weiter umgesetzt wird.
¹H-NMR(ppm in d⁶-DMSO): 1.336 (tr, 3H); 3.391 (s, 3H); 3.529 (s, 3H); 4.360 (g, 2H); 6.380 (tr, 1H); 8.218 (d, 1H); 8.778 (d, 1H); 14. 234 (s, 8H).

Gemäß Beispiel (II-1) bzw. entsprechend den allgemeinen Verfahrensangaben werden die folgenden Ausgangsprodukte der Formel (II) erhalten:

### Beispiel (II-2)

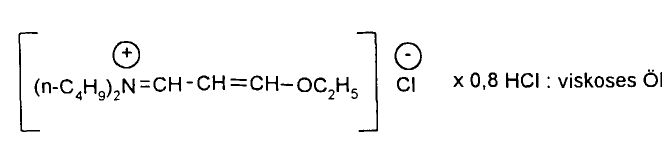

### Beispiel (II-3)

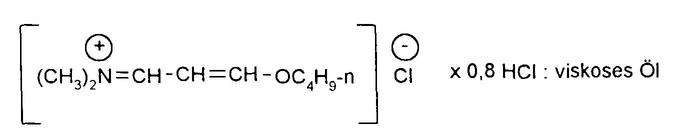

## Patentansprüche

1. Verfahren zur Herstellung von Pyrazolen der Formel (I) 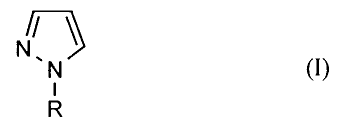 in welcher
R für Wasserstoff, geradkettiges oder verzweigtes C₁-C₄-Alkyl, gegebenenfalls einfach bis dreifach, gleich oder verschieden substituiertes C₃-C₆-Cycloalkyl, wobei als Substituenten Halogen, jeweils geradkettiges oder verzweigtes C₁-C₄-Alkyl, C₁-C₄-Alkoxy, C₁-C₄-Halogenalkyl und C₁-C₄-Halogenalkyloxy in Frage kommen, oder gegebenenfalls einfach bis dreifach, gleich oder verschieden substituiertes Phenyl oder Benzyl, wobei jeweils als Substituenten Halogen, Cyano, Nitro, jeweils geradkettiges oder verzweigtes C₁-C₄-Alkyl, C₁-C₄-Alkoxy, C₁-C₄-Alkylthio, C₁-C₄-Halogenalkyl, C₁-C₄-Ilalogenalkoxy und C₁-C₄-Halogenalkylthio (mit jeweils 1 bis 9 gleichen oder verschiedenen Halogenatomen)
steht,
dadurch gekennzeichnet, daß man Propenylidenammoniumchlorid-Derivate der Formel (II) 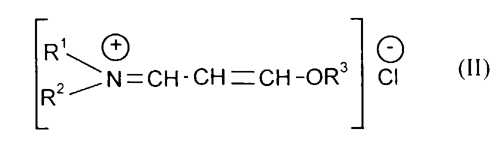 in welcher
R¹ und R² gleich oder verschieden sind und für geradkettiges oder verzweigtes C₁-C₄-Alkyl stehen und
R³ für geradkettiges oder verzweigtes C₁-C₄-Alkyl steht,
mit Hydrazinen der Formel (III)
H₂N-NH-R (III)
in welcher
R die oben angegebene Bedeutung hat,
in Gegenwart von Alkalimetall- oder Erdalkalimetall-hydroxiden, -amiden, -alkoholaten, -carbonaten, -hydrogencarbonaten, Trialkylaminen oder Dialkylaminen und in Gegenwart von Alkoholen, Ethern, gegebenenfalls halogenierten Kohlenwasserstoffen oder Wasser als Verdünnungsmittel umsetzt.

## Claims

1. Process for the preparation of pyrazoles of the formula (I) 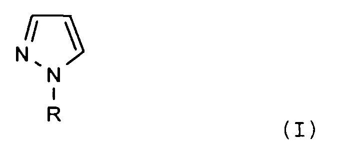 in which
R represents hydrogen, unbranched or branched C₁-C₄-alkyl, C₃-C₆-cycloalkyl which may optionally be identically or differently monosubstituted to trisubstituted, with the substituents being selected from the group consisting of halogen, C₁-C₄-alkyl, C₁-C₄-alkoxy, C₁-C₄-halogenoalkyl and C₁-C₄-halogenoalkyloxy, each of which may be unbranched or branched, and phenyl or benzyl which may optionally be identically or differently monosubstituted to trisubstituted, with in each case as substituents halogen, cyano, nitro, C₁-C₄-alkyl, C₁-C₄-alkoxy, C₁-C₄-alkylthio, C₁-C₄-halogenoalkyl, C₁-C₄-halogenoalkoxy and C₁-C₄-halogenoalkylthio (each having 1 to 9 identical or different halogen atoms) and each of which is unbranched or branched,
characterized in that propenylideneammonium chloride derivatives of the formula (II) 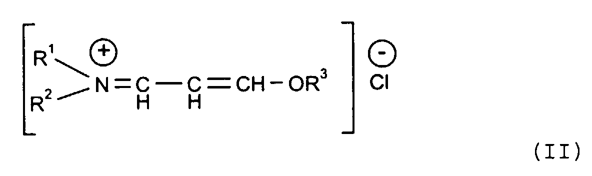 in which
R¹ and R² are identical or different and represent unbranched or branched C₁-C₄-alkyl and
R³ represents unbranched or branched C₁-C₄-alkyl,
are reacted with hydrazines of the formula (III)
H₂N-NH-R (III)
in which
R has the meaning given above,
in the presence of alkali metal hydroxides or alkaline earth metal hydroxides, alkali metal amides or alkaline earth metal amides, alkali metal alkoxides or alkaline earth metal alkoxides, alkali metal carbonates or alkaline earth metal carbonates, alkali metal hydrogen carbonates or alkaline earth metal hydrogen carbonates, trialkylamines or dialkylamines and in the presence of alchohols, ethers, halogenated or non-halogenated hydrocarbons or water as diluent.

## Revendications

1. Procédé de préparation de pyrazoles de formule (I) 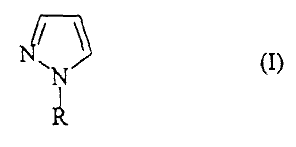 où
R représente l'hydrogène, alkyle en C₁-C₄ linéaire ou ramifié, cycloalkyle en C₃-C₆ éventuellement substitué une à trois fois de manière identique ou différente, les substituants pouvant être halogène, alkyle en C₁-C₄, alcoxy en C₁-C₄, halogénoalkyle en C₁-C₄ et halogénoalkyloxy en C₁-C₄ linéaires ou ramifiés, ou phényle ou benzyle éventuellement substitué une à trois fois de manière identique ou différente, les substituants pouvant être dans chaque cas halogène, cyano, nitro, alkyle en C₁-C₄, alcoxy en C₁-C₄, alkylthio en C₁-C₄, halogénoalkyle en C₁-C₄, halogénoalcoxy en C₁-C₄ et halogénoalkylthio en C₁-C₄ linéaires ou ramifiés (avec dans chaque cas 1 à 9 atomes d'halogène identiques ou différents),
caractérisé en ce que l'on fait réagir des dérivés de chlorure de propénylidèneaminonium de formule (II) 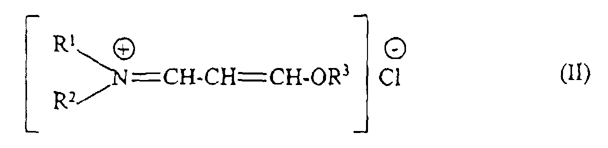 où
R¹ et R² sont identiques ou différents et représentent alkyle en C₁-C₄ linéaire ou ramifié et
R³ représente alkyle en C₁-C₄ linéaire ou ramifié,
avec des hydrazines de formule (III)
H₂N-NH-R (III)
où
R a la signification indiquée ci-dessus,
en présence d'hydroxydes, amides, alcoolates, carbonates, hydrogénocarbonates alcalins ou alcalino-terreux, de trialkylamines ou de dialkylamines et en présence d'alcools, d'éthers, d'hydrocarbures éventuellement halogénés ou d'eau comme diluant.
